# EUROPEAN PATENT SPECIFICATION

(11) **EP 2 185 081 B1**
(45) Date of publication and mention of the grant of the patent: **11.09.2013**
(21) Application number: 08786339.5
(22) Date of filing: 23.07.2008
(51) Int. Cl.: A61B 17/04, A61B 17/064, A61B 17/86

(54) **BONE REPAIR EYELET**
KNOCHENREPARATUR-ÖSE
OEILLET DE RÉPARATION DES OS

(30) Priority: 25.07.2007 EP 07113090
(43) Date of publication of application: 19.05.2010
(73) Proprietor: Bless, Nicolas, 5222 Umiken (CH)
(72) Inventor: Bless, Nicolas, 5222 Umiken (CH)
(74) Representative: Bohest AG
(86) International application number: PCT/EP2008/059632
(87) International publication number: WO 2009/013304

(56) References cited:
- EP-A- 1 344 495
- WO-A-02/053039
- GB-A- 1 306 429
- US-A- 5 306 290
- US-A- 6 099 568
- US-A1- 2002 133 179
- US-A1- 2003 135 225
- US-A1- 2004 199 166
- US-A1- 2006 276 871
- US-A1- 2007 049 944

## Description

The present invention deals with a bone repair eyelet according to claim 1 and a bone repair kit according to claim 4.

When a person falls on his/her shoulder the ligaments (*ligamentum acromioclaviculare, ligamentum trapezoidum, ligamentum conoideum*) between the *scapula* and the *clavicula* may become damaged (e.g. ruptured or partially ruptured). The tension of strong muscles in that area result in that the lateral outer end of the *clavicula* is moved in a cranial direction relative to the *scapula.* Through surgery the anatomically correct positions of the *scapula* and the *clavicula* relative to one another must be reestablished, and this anatomically correct relative position of the *scapula* and the *clavicula* must then be securely maintained for some weeks in order to allow the ligaments to heal or recover.

Up to now, different methods are used for stabilisation of the bones relative to one another. In a first known method a percutaneous Kirschner wire is introduced through the *acromion* into the *clavicula.* This connection is sometimes additionally supported by a metal *cerclage.* The disadvantages of this method are the possibility of a dislocation of the connecting rod and the need to surgically expose the *articulatio acromioclavicularis* (the joint between the *acromion* and the *clavicula)* in order to be able to adequately fix the cerclage.

In a further known method a so-called "Bosworth"-screw is screwed through the *clavicula* into the *processus coracoideus* of the *scapula.* However, the bone is weakened due to the large diameter of the screw, and in some cases a secondary loosening of the screw may occur and, as a consequence, stability of the anatomically correct realtive positions of the bones is no longer maintained.

Still a further method suggests the fixation of one or more bone plates in order to fix the bones in the desired positions relative to one another. This is a very reliable method for maintaining the desired relative position of the bones during the healing/recovery period. However, the fixation of bone plates suffers from the disadvantage that they are typically accompanied by damages to the surrounding soft tissue.

Yet another method suggests that chords made of a resorbable material are wound around the *processus coracoideus* and the *clavicula* in order to maintain the desired relative position of these bones, however, similar to the plates an extensive exposure with mandatory damage to the soft tissue is necessary in order to fixate the chords around the bone.

Finally, it has been suggested that a bone anchor be fixed to the *processus coracoideus* and that a suture connected to the bone anchor then be wound around the *clavicula*. However, this does again require exposure of the *clavicula* to a substantial extent, which is inconvenient for the patient.

Bone anchors with sutures connected to them are used together with bone eyelets in the surgical treatment of urinary incontinence, as is known from US 6,575,987 (see

Fig. 33, for example). The eyelet disclosed there has a hollow shaft and a rod arranged in the proximal end portion of the hollow shaft. The rod extends from a location on the inner wall of the hollow shaft across the center of the shaft to an oppositely arranged location of the inner wall. The two ends of the suture connected to the bone anchor are guided through the hollow shaft at different sides of the rod. The eyelet can then be introduced into the bone through an axial movement, whereupon the two ends of the suture can be knotted immediately above the crossbeam.

While the bone anchor and eyelet disclosed in US 6,575,987 allows to reduce damage to the soft tissue, it suffers from the disadvantage that it can only be introduced into the bone through an axial movement and that is does not have a thread or similar anchoring structure on the outer wall of its shaft. In addition, rotational introduction of the eyelet shown in Fig. 33 of US 6,575,987 (see Fig. 32) would cause the sutures extending along both sides of the rod to get twisted.

The fastening device of the blind type for permanent attachment to a support panel disclosed in GB-A-1 306 429 comprises a support member with one opening in the center and a deformable body member. It consists of two separate parts which must be assembled. Furthermore, there is no space between the two parts limiting the guidance for the suture to a single opening. This fastening device is neither suitable nor suggested for use as a bone anchor.

The graft ligament anchor disclosed in WO-A-00/47137 includes a tubular body bore with a deformable wall disposed therein. An expansion device is configured for insertion into the tubular body bore. Similar to the device of the already discussed GB-A-1 306 429 two separate parts have to be assembled in order to establish the mechanical function of the anchor, thus making the device difficult to handle. Similar considerations hold for the device of US 2002 / 0133179.

The fixing device disclosed in WO-A-02/053039 has a shell-shaped structure in order to withstand compressive loading and to stay in place with force applied in a first longitudinal direction of the device. The device has only one central opening forming the only passage allowing a suture to be passed therethrough. The suture can then be knotted to prevent it from slipping back through that central opening. No means are provided for making the device withstand forces applied in the direction opposite to the first longitudinal direction.In US 5,306,290 a frusto-conical button with at least one and preferably at least a pair of bottom holes is illustrated. Additional side holes are provided. Due to the sloped surface the button can withstand compressive loading and stay in place with force applied in a first longitudinal direction of the device. After the frusto-conical button in US 5,306,290 has been fixed to the bone, it is virtually impossible to fix a suture that has not been previously guided through the bottom or side holes. US-A-2007/0049944 shows an anchor member including a body portion with an internal passage and a collar arranged in that internal passage which includes a first and a second passage. However, rotational introduction of the anchor member causes the sutures to get twisted. Similar considerations hold for the device shown in US 6 099 568.

In US 2004/0199166 a button implant is illustrated. This implant includes a ripped, cannulated body with a central cannula extending through the implant and an angled head. Two openings formed through the head, one on either side of the head opening of the cannula, accommodate suture strands. However, once the implant has been applied it is very difficult to fix a suture introduced through the central cannula through knotting of the suture.

The present invention overcomes the disadvantages of the prior art devices and suggests a bone repair eyelet as it is characterised by the features of independent claim 1. Advantageous embodiments are subject of the dependent claims.

In particular, the bone repair eyelet according to the invention comprises a single piece that has a hollow shaft and two rods that extend from the inner wall of the hollow shaft to a ring. The ring has an opening located in the center of the shaft that extends in the longitudinal direction of the shaft, so that the hollow shaft is diviced into three passages which are separated from one another.

The rods may be of variable size and profile and extend to a ring of variable size and profile with the opening of the ring being located in the center of the shaft. Thus, the hollow shaft is divided into three separate spaces. The rods and the ring form both a bearing for the sutures knotted above them and an abutment for the screw driver used to apply the eyelet.

Prior to introduction of the eyelet into the bone, the sutures are guided through the opening provided in the ring . Since the opening of the ring is located in the center of the shaft, the eyelet can be rotated during introduction into the bone, with rotation having no impact on the sutures. In particular, the sutures do not get twisted then. This simplifies introduction of the eyelet for the surgeon, and in particular allows also an eyelet having a thread or similar structure on the outer wall of its shaft to be screwed into the bone. Fixation of the two bones in a desired position relative to one another only requires a bone anchor, a bone eyelet as described, and a suture. The bone anchor can be introduced into the underlying bone through a bore that is to be created for introduction of the eyelet, so that damages to the soft tissue can be kept at a minimum when compared to many of the conventional methods (see further above).

As already mentioned, an advantageous embodiment of the eyelet according to the invention comprises a supporting edge at one end (e.g. the proximal end) which extends outwardly from the hollow shaft. This supporting edge forms an abutment indicating to the surgeon that the eyelet cannot be introduced further into the bone.

As already mentioned, in a particularly advantageous embodiment of the eyelet according to the invention the outer wall of the hollow shaft is provided with a thread. This allows the surgeon to screw the eyelet into the bone without twisting the sutures, thus making handling more comfortable for the surgeon, while the thread provides for a better anchoring of the eyelet in the bone.

The eyelet can be made of a biocompatible material such as titanium, however, bio-resorbable materials may also be contemplated.

Another aspect of the invention is related to a bone repair kit comprising one or more eyelets as described above. In particular, the kit may comprise two or more such eyelets which could be connected to one another by sutures in order to maintain two bones in a desired position relative to one another.

In one embodiment of the bone repair kit according to the invention, the kit comprises a bone anchor different from the one or more eyelets. For example, the bone anchor may be a simple conventional bone anchor which can be easily introduced into an underlying bone through the hole which is to be created in the bone into which the eyelet is to be introduced.

A particular advantageous embodiment of the bone repair kit further comprises a screw driver having a hollow shaft. The wall of the hollow shaft of the screw driver at its end dedicated to engage the eyelet is provided with slits extending in the longitudinal direction of the hollow shaft. The slits are arranged in a manner corresponding to the arrangement of the rods of the shaft of the eyelet. The outer diameter of the hollow shaft of the screw driver at least in its portion dedicated to engage the eyelet is chosen such that this end of the screw driver can be introduced into the hollow shaft of the eyelet. The width of the rods of the eyelet and the width of the slits in the wall of the portion of the screw driver dedicated to engage the eyelet are chosen such that the rods of the eyelet can be introduced into the slits of the screw driver as the screw driver is introduced into the hollow shaft of the eyelet.

This embodiment of the kit provides for a very convenient handling of the eyelet through the surgeon. Introduction of the eyelet into the bone can be performed as follows: Firstly, the sutures connected to the bone anchor or to a further eyelet already fixed to the other bone are guided through the centrally located opening in the ring between the rods of the eyelet. The sutures are then guided through the hollow shaft of the screw driver. Next, the one end of the screw driver is axially inserted into the hollow shaft of the eyelet, with the rods of the eyelet being introduced into the correspondingly arranged slits of the screw driver. Upon screwing the eyelet into the bone, the wall portions of the screw driver defining the slits engage the rods of the eyelet and rotate the eyelet without twisting the sutures. Once the eyelet has been completely screwed into the bone, the screw driver is removed and the ends of the sutures are knotted immediately above the ring or one of the rods.

Further advantageous aspects of the invention will become apparent from the following descripition of an embodiment with the aid of the drawings in which:
- Fig. 1: shows three different types of damages to the shoulder,
- Fig. 2: shows a cross-sectional view of an embodiment of the bone repair eyelet according to the invention,
- Fig. 3: shows a top view of the embodiment of the eyelet of Fig. 2,
- Fig. 4: shows a perspective view of an embodiment of the screw driver of the bone repair kit according to the invention,
- Fig. 5: shows an exploded view of the parts of one embodiment of the bone repair kit according to the invention,
- Fig. 6: shows the eyelet of Fig. 2 with a suture to be knotted above one rod of the eyelet,
- Fig. 7: shows eyelet of Fig. 6 with the knotted suture,
- Fig. 8: shows an application of a kit comprising a bone anchor and an eyelet, with the bone anchor and the bone eyelet being already fixed to the bones,
- Fig. 9: shows another application of a kit comprising two eyelets, with the eyelets being already fixed to the bones, and
- Fig. 10: shows the shoulder of a patient with several eyelets being fixed to various bones of the shoulder.

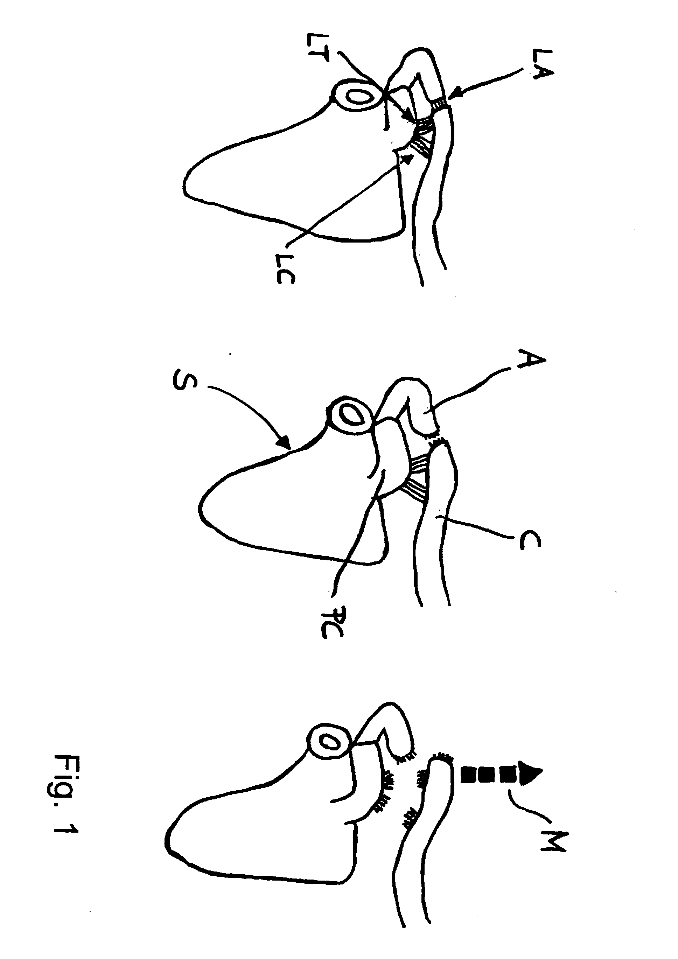

In Fig. 1 three different types of damages to the shoulder of a patient are shown, which are classified according to Tossy as "Tossy I" (left hand side in Fig. 1), "Tossy II" (centrally in Fig. 1) and "Tossy III" (right hand side in Fig. 1). The various bones *acromion* A, the *clavicula* C, the *processus coracoideus* PC and the *scapula* S can be seen in Fig. 1 as well as the various ligaments.

In the type of damage "Tossy I" shown on the left in Fig. 1 the *ligamentum acromioclaviulare* LA, the *ligamentum trapezoidum* LT and the *ligamentum conoideum* LC are extended but not ruptured. In the type of damage "Tossy II" shown centrally in Fig. 1 the *ligamentum acromioclaviculare* LA is ruptured while the other ligaments are only extended but not ruptured. The *clavicula* is slightly moved upwards due to the force exerted on the *clavicula* by muscles (not shown). In the type of damage "Tossy III" shown on the right in Fig. 1 all ligaments LA,LT,LC are ruptured, and the *clavicula* is substantially moved upwards due to the force exterted on the *clavicula* by muscles (represented by the arrow M). While for the type "Tossy I" and "Tossy II" it may not be necessary to surgically intervene, for the "Tossy III" type this is required in order to reposition the bones to their anatomically correct relative position so that the ligaments may heal and/or recover.

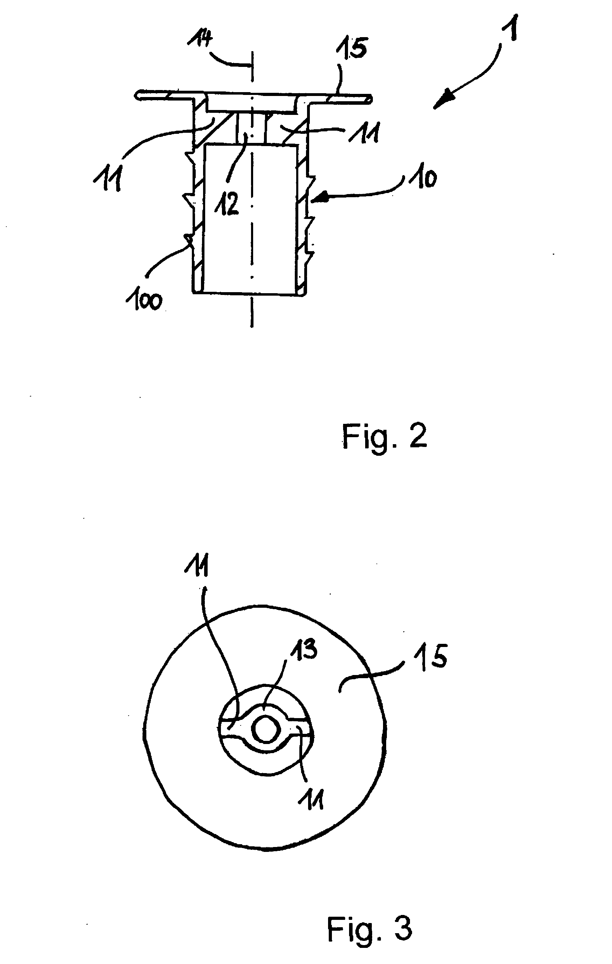

In Fig. 2 and Fig. 3 an embodiment of the eyelet 1 according to the invention is shown. Eyelet 1 comprises a hollow shaft 10 with two rods 11 extending from the inner wall of hollow shaft 10 to a ring 13 having an opening 12 located in the center of the hollow shaft 10. The opening 12 of the ring 13 is extending in the longitudinal direction represented by axis 14 in Fig. 2 of hollow shaft 10. Hollow shaft 10 is provided with a thread 100 on its outer wall. Eyelet 1 further comprises a supporting edge 15 at one end which extends outwardly from hollow shaft 10.

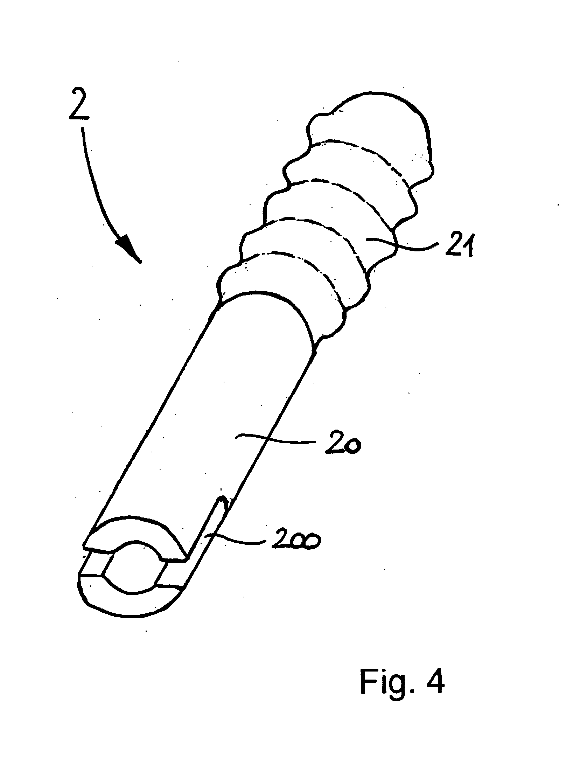

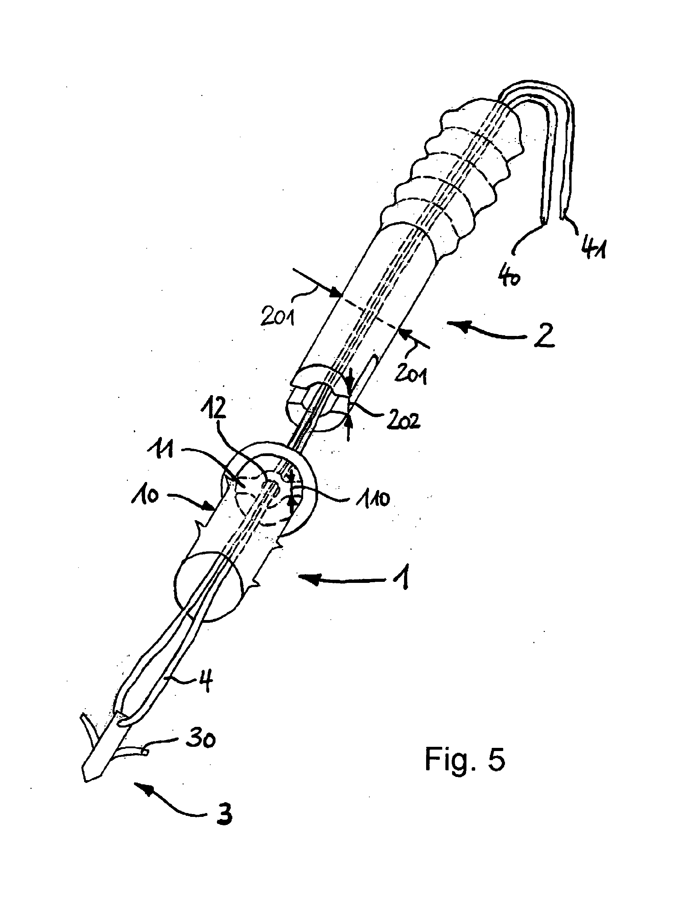

In Fig. 4 an embodiment of the screw driver 2 of a bone repair kit according to the invention is shown. Screw driver 2 has a hollow shaft 20 which is provided at its end dedicated to engage the eyelet 1 with two slits 200 extending in the longitudinal direction of hollow shaft 20. Screw driver 2 is further provided with an engagement or gripping portion 21 at the other end, where either an actuation instrument may engage screw driver 2 or where the surgeon may grab the screw driver. The screw driver is hollow along its entire length, as will be discussed further below with reference to Fig. 5.

Fig. 5 shows an exploded view of the parts of one embodiment of the bone repair kit according to the invention. This embodiment of the kit comprises a conventional bone anchor 3, an eyelet 1 as described above, a screw driver 2 as described above, and a suture 4. Bone anchor 3 has two arms 30 which can be moved towards the body of bone anchor 3 and which can spread apart as shown in Fig. 5. Suture 4 is guided through a hole in bone anchor 3 and is further guided through hollow shaft 10 and opening 12 in the ring 13 provided between the rods 11 of eyelet 1, and is still further guided through hollow screw driver 2 so that the two ends 40,41 of suture 4 project out of the proximal end of screw driver 2.

The outer diameter 201 of the shaft 20 of screw driver 2 at its end dedicated to engage the eyelet 1 is such that this end can be introduced into hollow shaft 10 of eyelet 1. The width 202 of the slits 20 of screw driver 2 and the width 110 of the rods 11 of eyelet 1 are chosen such, that the rods 11 of eyelet 1 can be introduced into the slits 20 of screw driver 2 as the screw driver 2 is introduced into the hollow shaft 10 of eyelet 1.

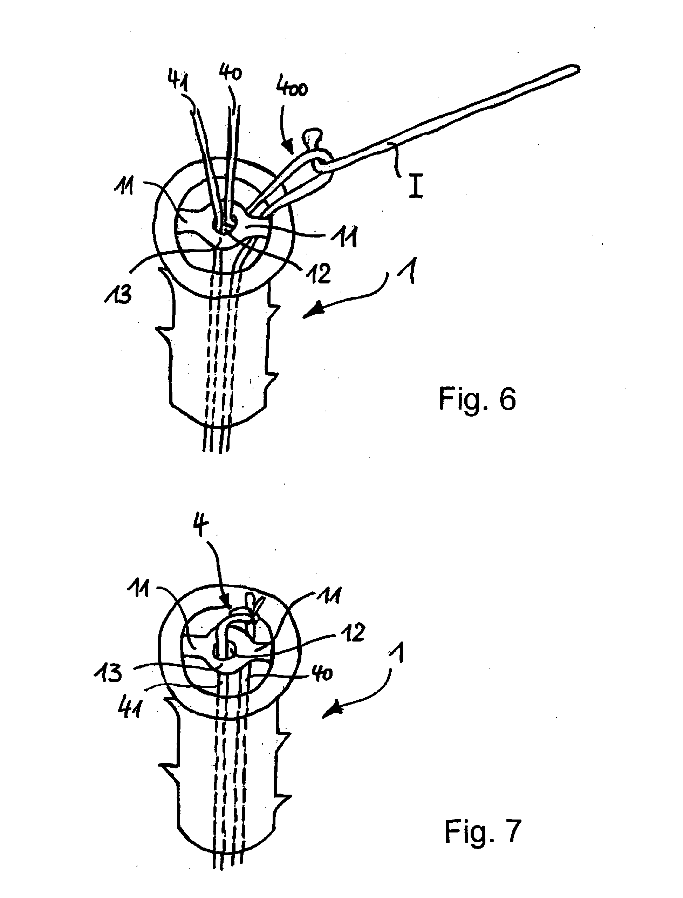

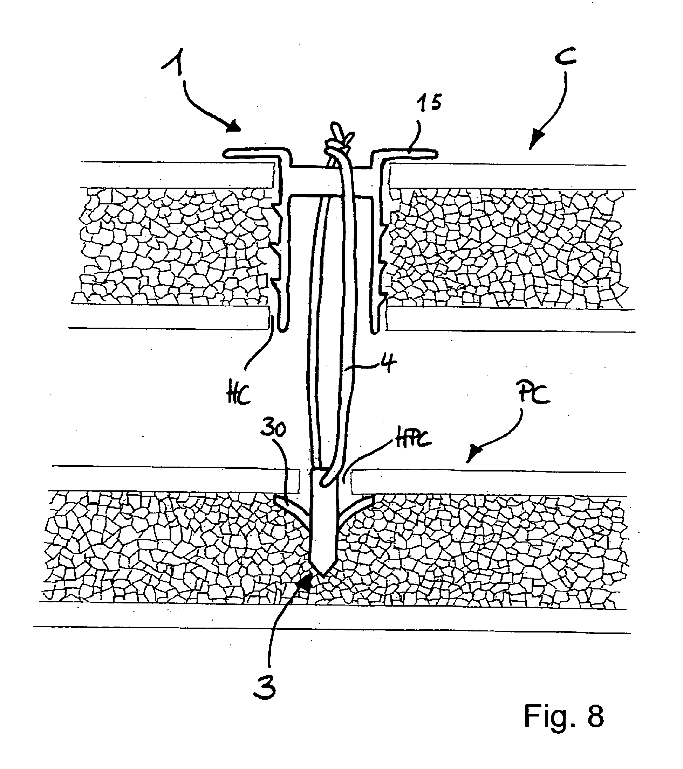

One application of a kit comprising bone anchor 3, eyelet 1, suture 4 and screw driver 2 will be explained with the aid of Fig. 6, Fig. 7 and Fig. 8. In Fig. 8 two closely arranged bones, such as for example the *clavicula* C and the *processus coracoideus* PC, are shown. In order to stabilise the relative position of these bones, first of all a small incision must be made in the skin of the patient and a comparatively small portion of the *clavicula* C must be exposed. A hole HC is then to be formed through the *clavicula* C having a diameter which must be suitable to allow eyelet 1 to be screwed into *clavicula* C at a later time. After hole HC has been formed in *clavicula* C, a smaller hole HPC must be formed through the *corticalis* (cortical bone) of the *processus coracoideus* PC. Bone anchor 3 with together with suture 4 (see Fig. 5) is then inserted through holes HC and HPC into the *spongiosa* (cancellous bone) of the *processus coracoideus* PC. Arms 30 of bone anchor 3 are spread and thus bone anchor 3 cannot be retracted from the *processus coracoideus* PC after having been inserted. Next, eyelet 1 is screwed into hole HC formed in the *clavicula* C with the aid of screw driver 2. When screwing eyelet 1 into hole HC, the walls of the screw driver 2 defining the slits 20 engage rods 11 while the suture 4 extends through hole 12 in the ring 13 of eyelet 1, so that eyelet 1 can be screwed into hole HC without the suture 4 getting twisted (see also Fig. 5). The thread 100 provided on the outer wall of eyelet 1 provides for a good anchoring of eyelet 1 in the *clavicula* C. Once eyelet 1 has been fully screwed into hole HC such that supporting edge 15 abuts against *clavicula* C, the screw driver 2 is removed and the two ends 40,41 of suture 4 project out of opening 12 of eyelet 1 and must be knotted.

This can be performed in a convenient manner as can be seen in Fig. 6. The surgeon or an assistant can form a loop 400 with the aid of a hook-like instrument I, and the two ends 40,41 of suture 4 can then be knotted with loop 400 above rods 11 or ring 13 of eyelet 1. The eyelet 1 with the thus knotted suture 4 is shown in Fig. 7.

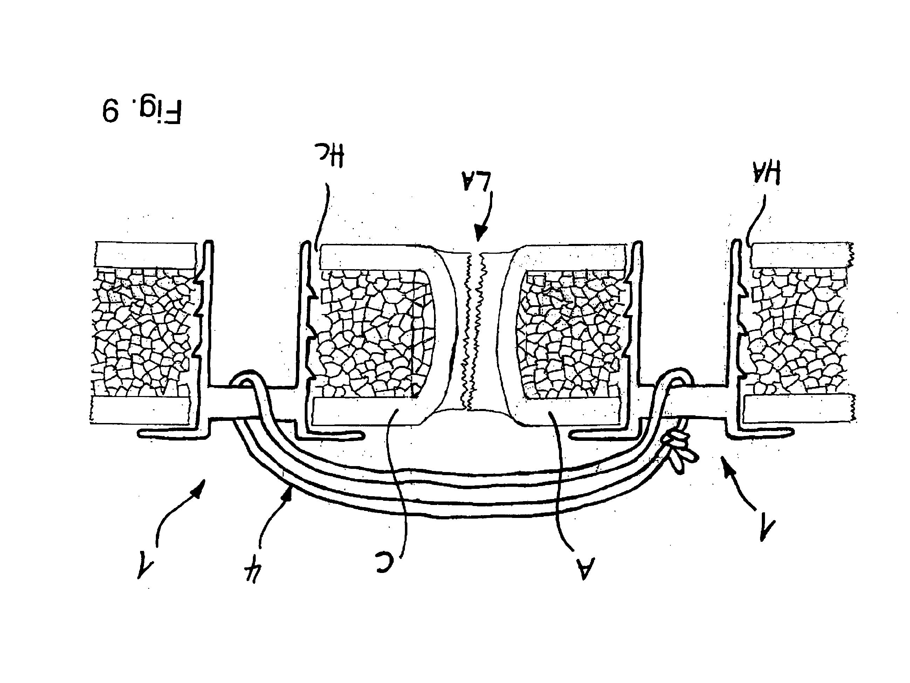

In Fig. 9 another application of a further kit comprising two eyelets 1 instead of one eyelet 1 and a bone anchor 3 is shown. For example, the bones in this application may be the *clavicula* C and the *acromion* A with the *ligamentum acromioclaviculare* LA being ruptured. The suture 4 may first be guided through the opening 12 in the ring 13 between the rods 11 of eyelet 1. The surgeon must form hole HC in the *clavicula* C and screw eyelet 1 into *clavicula* C with the aid of screw driver 2 as this has been described above. However, only a small skin incision must be then made at the *acromion* A and a hole HA must be formed in the *acromion* A. It is not necessary to surgically expose the entire area between the two holes HC and HA. Rather, the two ends of the suture 4 can be guided from hole HC to hole HA under the skin with the aid of a needle. The manner how the suture 4 is knotted at eyelet 1 which has been screwed into hole HA of *acromion* A is similar to that described above. Surgical exposure is thus kept at a minimum.

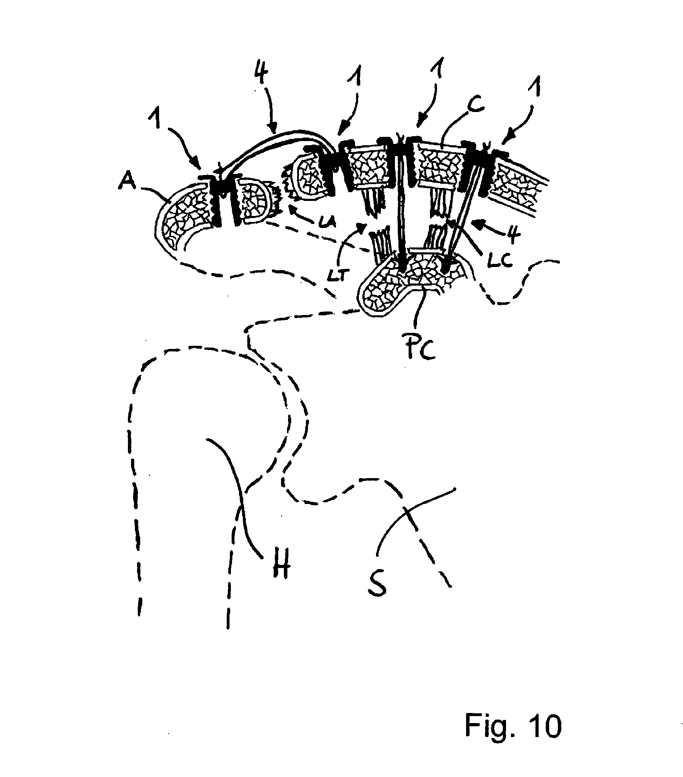

A schematic detail of an entire shoulder is shown in Fig. 10 with various eyelets 1, bone anchors 3 and sutures 4 having been inserted. In this case all three ligaments, the *ligamentum acromioclaviculare* LA, the *ligamentum trapezoidum* LT, and the *ligamentum conoideum* LC are ruptured. For stabilising the position of the *clavicula* C relative to the *acromion* A, two eyelets 1 are connected by a suture 4 (see also Fig. 9 and the corresponding description above). Stabilisation of the position of the *clavicula* C relative to the *processus coracoideus* PC, however, is performed with two eyelets 1 and two bone anchors 3 and corresponding sutures 4, respectively. The *scapula* S and the *humerus* H are shown for the sake of completeness.

While embodiments for the application of the eyelet and the kit according to the invention have been explicitly shown for the shoulder, other fields of application are well conceivable, for example in the region of the distal tibiofibular joint, etc.. It goes without saying, that the diameter of the eyelet, the size of the various passages through the eyelet and the size of the supporting edge of the eyelet can be varied depending on the respective application or requirements.

## Claims

1. Bone repair eyelet (1) comprising a single piece having a hollow shaft (10) and two rods (11) extending from the inner wall of the hollow shaft (10) to a ring (13) having an opening (12) located in the center of the shaft (10) and extending in the longitudinal direction (A) of the shaft (10), so that the hollow shaft is divided into three longitudinal passages which are separated from one another.

2. Eyelet according to claim 1, further comprising a supporting edge (15) at one end which extends outwardly from the hollow shaft (10).

3. Eyelet according to any one of the preceding claims, wherein the outer wall of the hollow shaft (10) is provided with a thread (100).

4. Bone repair kit comprising one or more eyelets (1) according to any one of the preceding claims.

5. Bone repair kit according to claim 4, further comprising one or more bone anchors (3) different from the one or more eyelets (1).

6. Bone repair kit according to any one of claims 4 or 5, further comprising a screw driver (2) having a hollow shaft (20), the wall of the hollow shaft (20) of the screw driver at its end dedicated to engage the eyelet being provided with slits (200) extending
in the longitudinal direction of the hollow shaft (20) and being arranged in a manner corresponding to the arrangement of the rods of the shaft (10) of the eyelet (1), wherein the outer diameter (201) of the hollow shaft (20) of the screw driver (2) at least in its portion dedicated to engage the eyelet being chosen such that this end of the screw driver (2) can be introduced into the hollow shaft (10) of the eyelet (1), and wherein the width (110) of the rods (11) of the eyelet (1) and the width (202) of the slits (200) in the wall of the portion of the screw driver (2) dedicated to engage the eyelet (1) being chosen such that the rods (11) of the eyelet (1) may be introduced into the slits (200) of the screw driver (2) as it is introduced into the hollow shaft (10) of the eyelet (1).

## Patentansprüche

1. Knochenreparatur-Öse (1) umfassend ein einziges Teil mit einem hohlen Schaft (10) und zwei Streben (11), die sich von der Innenwand des hohlen Schafts (10) hin zu einem Ring (13) mit einer in der Mitte des Schafts (10) angeordneten und sich in der Längsrichtung (A) des Schafts (10) erstreckenden Öffnung (12) erstrecken, so dass der hohle Schaft in drei voneinander getrennte longitudinale Durchgänge unterteilt ist.

2. Öse nach Anspruch 1, ferner umfassend einen Auflagerand (15) an einem Ende, welcher sich von dem hohlen Schaft (10) weg nach aussen erstreckt.

3. Öse nach einem der vorangehenden Ansprüche, wobei die Aussenwand des hohlen Schafts (10) mit einem Gewinde (100) versehen ist.

4. Knochenreparatur-Kit, umfassend eine oder mehrere Ösen (1) gemäss einem der vorangehenden Ansprüche.

5. Knochenreparatur-Kit nach Anspruch 4, ferner umfassend einen oder mehrere Knochenanker (3), die von den einen oder mehreren Ösen (1) verschieden sind.

6. Knochenreparatur-Kit nach einem der Ansprüche 4 oder 5, ferner umfassend einen Schraubendreher (2) mit einem hohlen Schaft (20), wobei die Wand des hohlen Schafts (20) des Schraubendrehers an dessen Ende, das zum Eingriff mit der Öse bestimmt ist, mit Schlitzen (200) versehen ist, die sich in der Längsrichtung des hohlen Schafts (20) erstrecken und in einer zur Anordnung der Streben des Schafts (10) der Öse (1) korrespondierenden Weise angeordnet sind, wobei der Außendurchmesser ( 201) des hohlen Schafts (20) des Schraubendrehers (2) zumindest in seinem Abschnitt, der zum Eingriff mit der Öse bestimmt ist, derart gewählt ist, dass dieses Ende des Schraubendrehers (2) in den hohlen Schaft (10) der Öse (1) eingebracht werden kann, und wobei die Breite (110) der Streben (11) der Öse (1) und die Breite (202) der Schlitze (200) in der Wand des Abschnitts des Schraubendrehers (2), der dazu bestimmt ist, in die Öse (1) einzugreifen, derart gewählt ist, dass die Streben (11) der Öse (1) in die Schlitze (200) des Schraubendrehers (2) eingeführt werden können während dieser in den hohlen Schaft (10) der Öse (1) eingeführt wird.

## Revendications

1. OEillet pour réparation osseuse (1) comprenant une pièce simple ayant un axe creux (10) et deux tiges (11) s'étendant depuis la paroi intérieure de l'axe creux (10) jusqu'à une bague (13) comportant une ouverture (12) située au centre de l'axe (10) et s'étendant dans la direction longitudinale (A) de l'axe (10), de sorte que l'axe creux est divisé en trois passages longitudinaux qui sont séparés les uns des autres.

2. OEillet selon la revendication 1, comprenant en outre un bord de support (15) à une extrémité qui s'étend vers l'extérieur depuis l'axe creux (10).

3. OEillet selon l'une quelconque des revendications précédentes, dans lequel la paroi extérieure de l'axe creux (10) est pourvue d'un filetage (100).

4. Nécessaire de réparation osseuse comprenant un ou plusieurs oeillets (1) selon l'une quelconque des revendications précédentes.

5. Nécessaire de réparation osseuse selon la revendication 4, comprenant en outre une ou plusieurs ancres à os (3) différentes desdits un ou plusieurs oeillets (1).

6. Nécessaire de réparation osseuse selon l'une quelconque des revendications 4 et 5, comprenant en outre un tournevis (2) ayant un axe creux (20), la paroi de l'axe creux (20) du tournevis à son extrémité prévue pour se mettre en prise avec l'oeillet étant pourvue de fentes (200) s'étendant dans la direction longitudinale de l'axe creux (20) et agencées d'une manière qui correspond à l'agencement des tiges de l'axe (10) de l'oeillet (1), dans lequel le diamètre extérieur (201) de l'axe creux (20) du tournevis (2) au moins dans sa partie prévue pour se mettre en prise avec l'oeillet étant choisi de telle manière que cette extrémité du tournevis (2) peut être introduite dans l'axe creux (10) de l'oeillet (1), et dans lequel la largeur (110) des tiges (11) de l'oeillet (1) et la largeur (202) des fentes (200) formées dans la paroi de la partie du tournevis (2) prévue pour se mettre en prise avec l'oeillet (1) étant choisies de telle manière que les tiges (11) de l'oeillet (1) peuvent être introduites dans les fentes (200) du tournevis (2) quand on introduit celui-ci dans l'axe creux (10) de l'oeillet (1).
